# EUROPÄISCHE PATENTSCHRIFT

(11) **EP 0 554 543 B1**
(45) Veröffentlichungstag und Bekanntmachung des Hinweises auf die Patenterteilung: **28.02.1996**
(21) Anmeldenummer: 92121028.2
(22) Anmeldetag: 10.12.1992
(51) Int. Cl.: C07C 271/28, A61K 31/27, C07C 275/40, C07C 233/43, A61K 31/17

(54) **Neue 1,2,4-Triaminobenzol-Derivate und Verfahren zu deren Herstellung**
1,2,4-Triaminobenzene derivatives and process for their preparation
Dérivés de 1,2,4-triaminobenzène et procédé de leur préparation

(30) Priorität: 08.01.1992 DE 4200259
(43) Veröffentlichungstag der Anmeldung: 11.08.1993
(73) Patentinhaber: ASTA Medica Aktiengesellschaft, D-01277 Dresden (DE)
(72) Erfinder: Dieter, Hans-Reinhold, Dr., W-6100 Darmstadt (DE); Engel, Jürgen, Prof., W-8755 Alzenau (DE); Kutscher, Bernhard, Dr., W-6457 Maintal 1 (DE); Polymeropoulos, Emmanuel, Dr., W-6000 Frankfurt am Main 1 (DE); Szelenyi, Stefan, Prof., W-8501 Schwaig (DE); Nickel, Bernd, Dr., W-6109 Mühltal 6 (DE)

(56) Entgegenhaltungen:
- EP-A- 0 343 429
- DE-A- 3 337 593
- CHEMIKER-ZEITUNG Bd. 103, Nr. 12, Dezember 1979, Heidelberg, DE; Seiten 387 - 399, W. VON BEBENBURG et al: "Substituierte Polyaminopyridine"

## Beschreibung

In der Zeitschrift Chemiker Zeitung 103 (1979) wird auf Seite 390 die Herstellung des 2-Carbethoxy-amino-5-(2,4,6-trimethylbenzylamino)-anilins beziehungsweise dessen Hydrochlorids (Verbindung 81) beschrieben. Es finden sich in dieser Stelle keinerlei Angaben über eine pharmakologische Wirkung dieser Verbindung.
Die Erfindung betrifft die durch die Patentansprüche definierten Gegenstände.

Die erfindungsgemäßen Verbindungen sind pharmakologisch wirksam. Insbesondere besitzen die erfindungsgemäßen Verbindungen antikonvulsive, antipyretische und muskelrelaxierende Wirkungen. Darüberhinaus besitzen sie auch eine spezifische periphere analgetische Wirkung.

Der Erfindung liegt also die Aufgabe zugrunde, Verbindungen mit günstigen pharmakologischen Eigenschaften zur Verfügung zu stellen, die beispielsweise als antiepileptische, muskelrelaxierende,
fiebersenkende sowie peripher analgetisch wirkende Arzneimittel verwertbar sind.
Die folgenden Erläuterungen stellen wichtige beispielhafte Angaben dar:
Die in der Formel I vorkommenden Alkylgruppen, Halogenalkylgruppen, Alkenylgrupen, Alkinylgruppen, Alkoxygruppen, Alkylaminogruppen, Alkanoylaminogruppen, Alkanoyloxyygruppen beziehungsweise ganz allgemein Alkanoylgruppen können gerade oder verzweigt sein. Dasselbe gilt auch für Alkyl- und Alkyloxygruppen (= Alkoxygruppen), falls diese Bestandteil anderer zusammengesetzter Gruppen sind (Zum Beispiel in Form einer Monoalkyl- oder Dialkylaminogruppe, Alkanoylaminogruppe, Alkoxycarbonylaminogruppe, Carbalkoxygruppe, Alkylcarbonylgruppe und analoge Gruppen. Bei der C₃-C₇-Cycloalkylgruppe handelt es sich vorzugsweise um Cyclopentyl oder Cyclohexyl. C₂-C₆-Alkenylbedeutet vorzugsweise Allyl. C₂-C₆-Alkinyl bedeutet vorzugsweise Propargyl.

Bei den Halogenatomen handelt es sich um Chlor, Brom oder Fluor, insbesondere Chlor und Fluor. Die Alkyl- und Alkoxygruppen als solche oder als Bestandteil von anderen zusammengesetzten Gruppen bestehen insbesondere aus 1-4 C-Atomen, vorzugsweise 1 oder 2 C-Atomen. Alkanoylgruppen, wie zum Beispiel Alkanoylaminogruppen oder Alkanoyloxygruppen bestehen insbesondere aus 2-4, vorzugsweise 2-3 C-Atomen. Alk besteht insbesondere aus 1-3, vorzugsweise 1 oder 2 C-Atomen.

Besonders günstige Eigenschaften haben solche Verbindungen der Formel I, worin R₁ Wasserstoff, C₁-C₆-Alkyl oder C₂-C₆-Alkanoyl, R₂ Wasserstoff oder C₁-C₆-Alkyl, R₄ und R₅ Wasserstoff, R₃ C₁-C₆-Alkoxy, die Alkylengruppe Alk direkt an das Stickstoffatom gebunden ist (n=0) und CH₂ bedeutet und Ar ein Halogenphenylrest vorzugsweise Fluor-phenyl (zum Beispiel 4-Fluor-phenyl) oder ein Trifluormethylrest (zum Beispiel 4-Trifluormethyl-phenyl) darstellt.

Diejenigen Verbindungen der Formel I, die asymmetrische Kohlenstoffatome enthalten und in der Regel als Razemate anfallen, können in an sich bekannter Weise beispielsweise mit Hilfe einer optisch aktiven Säure in die optisch aktiven Isomeren gespalten werden. Es ist aber auch möglich, von vornherein eine optisch aktive Ausgangssubstanz einzusetzen, wobei dann als Endprodukt eine entsprechende optisch aktive beziehungsweise diastereomere Form erhalten wird. Die vorliegende Erfindung umfaßt also auch die D- und L-Form wie auch die DL-Mischung für den Fall, daß in der Verbindung der Formel I ein asymmetrisches C-Atom vorkommt und für den Fall von 2 und mehr asymmetrischen C-Atomen ebenso die entsprechenden diasteromeren Formen.

Je nach den Verfahrensbedingungen und Ausgangsstoffen erhält man die Endstoffe der Formel I in freier Form oder in Form ihrer Salze. Die Salze der Endstoffe können in an sich bekannter Weise, beispielsweise mit Alkali oder Ionenaustauschern wieder in die Basen übergeführt werden. Von den letzteren lassen sich durch Umsetzung mit organischen oder anorganischen-Säuren, insbesondere solchen, die zur Bildung von therapeutisch verwendbaren Salzen geeignet sind, Salze gewinnen.

Die erfindungsgemäßen Verbindungen sind zur Herstellung pharmazeutischer Zusammensetzungen geeignet. Die pharmazeutischen Zusammensetzungen beziehungsweise Medikamente können eine oder mehrere der erfindungsgemäßen Verbindungen enthalten. Zur Herstellung der pharmazeutischen Zubereitungen können die üblichen Träger- und Hilfsstoffe verwendet werden.

### Zu dem Verfahren a) (Reduktion der Nitrogruppe)

Für die Reduktion entsprechend dem Verfahren a) hat sich als besonders geeignet die katalytische Hydrierung erwiesen. Als Katalysatoren kommen zum Beispiel in Frage: Raney-Nickel, Edelmetalle wie Palladium und Platin sowie Verbindungen davon, mit und ohne Träger, wie beispielsweise Bariumsulfat, Calciumsulfat und so weiter. Es empfiehlt sich, die Hydrierung der Nitrogrupppe bei Temperaturen zwischen 20 und 100 ^{o}C und einem Druck von ungefähr 1 bis 70 bar in einem Lösungsmittel vorzunehmen. Als Lösungsmittel eignen sich beispielsweise C₁-C₄-Alkanole, C₂-C₄-Diole und deren Niederalkylether, cyclische Ether wie Dioxan, Tetrahydrofuran, Methoxy-ethanol, Wasser, aromatische Kohlenwasserstoffe (Benzol, Toluole, Xylole) sowie Gemische dieser Mittel. Für die anschließende Isolierung der reduzierten Verbindungen kann es in manchen Fällen von Vorteil sein, wenn zu Beginn dem zu hydrierenden Gemisch Trockenmittel, wie wasserfreies Natrium- oder Magnesiumsulfat zugesetzt werden.

Die Reduktion kann aber auch mit nascierendem Wasserstoff beispielsweise Zink/Salzsäure, Zinn/Salzsäure, Eisen/Salzsäure oder mit Salzen des Schwefelwasserstoffs in Alkohol/Wasser bei 70 bis 120 °C oder mit aktiviertem Aluminium in wasserhaltigem Äther bei 20 bis 40 ^{o}C oder mit ZinnII-Chlorid/Salzsäure oder mit Ammoniumformiat durchgeführt werden.
Falls eine Ausgangsverbindung eingesetzt wird, die eine Oxogruppe enthält (zum Beispiel Alkylcarbonyl) kann es zweckmäßig sein, diese Oxogruppe durch übliche Acetalbildung (zum Beispiel in Form des Ethylenacetals) zu schützen. Dies gilt insbesondere für die katalytisdche Hydrierung.

Das so erhaltene Reaktionsprodukt wird zweckmäßig sofort in der anfallenden Reaktionsmischung mit einer Verbindung umgesetzt, die geeignet ist, ein Wasserstoffatom der durch die Reduktion erhaltenen Aminogruppe durch die Gruppe -COR₃ zu ersetzen, ohne daß die reduzierte Verbindung isoliert werden muß. Insbesondere gilt dies für den Fall der katalytischen Hydrierung. Selbstverständlich kann die durch Reduktion der Nitrogruppe erhaltene Verbindung auch isoliert werden und dann die -COR₃-Gruppe eingeführt werden. Die Einführung der -COR₃-Gruppe kann in der hierfür üblichen Weise mit den hierfür üblichen Reagenzien insbesondere Halogeniden der Formel Hal-COR₃ (Hal=Cl, Br, J) erfolgen. Beispiele für solche Reagenzien sind: Halogenameisensäure-C₁-C₆-alkylester (zum Beispiel Ethylester wie Chlor, Brom- oder Jodameisensäureethylester), -phenylester oder Hal-CO-OAr. Falls R₃ eine C₁-C₆-Alkylgruppe ist, kommen als Acylierungsmittel zum Beispiel die Halogenide (Chloride, Bromide, Jodide) oder Anhydride von C₁-C₆-Alkancarbonsäuren, C₂-C₆-Alkencarbonsäuren, C₂-C₆-Alkincarbonsäuren oder Säuren der Formel ArCO₂H in Frage. Weiterhin kommen als Acylierungsmittel Halogenide der folgenden Formel Hal-CO-NH₂, Hal-CO-NH-C₁-C₆-Alkyl, Hal-CO-N(C₁-C₆-Alkyl)₂, Hal-CO-NHAr in Frage, wenn der Rest R₃ eine der angegebenen Aminfunktionen hat.
Letztere Verbindungen können aber auch in hierfür üblicher Weise aus Verbindungen der Formel I, worin R₃ C₁-C₆-Alkoxy oder Phenoxy ist durch Umsetzung mit Ammoniak, C₁-C₆-Alkylaminen, C₁-C₆-Dialkylaminen oder Aminen NH₂-Ar erhalten werden.
Diese Umsetzungen erfolgen vorzugsweise unter Druck (10-50 bar) bei Temperaturen zwischen 0 bis 220 ^{o}C vorzugsweise 100 bis 200^{o} C in einem inerten Lösungs- oder Dispergiermittel. Als solche Mittel kommen zum Beispiel in Frage: niedere aliphatische Alkohole (1-6 C-Atome wie Propanol, Isopropanol, Butanol, Methoxyethanol), niedere aliphatische Ether (Diethylether, Diisopropylether), aromatische Kohlenwasserstoffe (Benzol, Toluol, Xylol), cyclische Ether (Dioxan, Tetrahydrofuran), Ester von niederen aliphatischen Carbonsäuren mit niederen aliphatischen Alkoholen, Amide und N-alkyl-substituierte Amide von aliphatischen C₁-C₄-Carbonsäuren (Dimethylformamid, Dimethylacetamid), C₁-C₆-Dialkylsulfone (Dimethylsulfon, Tetramethylensulfon), C₁-C₆-Dialkylsulfoxide (Dimethylsulfoxid) sowie weitere aprotische Mittel wie N-Methylpyrrolidon, Tetramethylharnstoff, Hexamethylphosphorsäuretriamid, Acetonnitril sowie Gemische dieser Mittel. Gegebenenfalls kann diese Acylierung auch ohne die Verwendung eines Lösungsbeziehungsweise Dispergierungsmittels erfolgen. Ohne ein solches Mittel erfolgt die Reaktion beispielsweise zwischen 0 und 200 ^{o}C vorzugsweise zwischen 100 und 200 ⁰C.

Da die freien Amine der Formel I, worin die Gruppe -COR₃ Wasserstoff ist, sauerstoffempfindlich sind, wird zweckmäßig unter Stickstoffatmosphäre gearbeitet.

Im allgemeinen werden die Reationskomponenten in molaren Mengen umgesetzt. Gegebenenfalls kann es jedoch zweckmäßig sein, eine Reaktionskomponente in leichtem Überschuß einzuzsetzen. Gegebenenfalls wird die Umsetzung in Gegenwart von basischen beziehungsweise säurebindenden Mitteln durchgeführt.

Als derartige Mittel kommen zum Beispiel in Frage: anorganische Kondensationsmittel wie Ammoniak, Alkali- oder Erdalkalihydroxide, Alkali- oder Erdalkalicarbonate oder organische Basen wie Pyridin, tertiäre Amine (Triethylamin), Piperidin, Alkalialkoholate, Alkaliacetate oder auch Triethylphosphat. Bei den Alkalimetallen handelt es sich insbesondere um Natrium oder Kalium. Es kann auch unter PHasen-Transfer-Bedingungen (das heißt unter Zusatz eines oder mehrerer langkettiger Amine wie einem Benzyltributylammonium-halogenid, einem Tetrabutyl-ammonium-halogenid oder Benzyl-triphenyl-phosphoniumchlorid) gearbeitet werden.
Insbesondere, wenn Halogenameisensäureester beziehungsweise Halogenameisensäure-Derivate eingesetzt werden, werden als basische Kondensationsmittel tertiäre Amine, Alkalihydroxide, Alkaliacetate oder Alkalihydrogencarbonate verwendet.

Die zusätzlich vorhandenen Aminogruppen können zum Beispiel eine übliche Amino-Schutzgruppe enthalten. Solche Schutzgruppen können nach Beendigung der Reaktion solvolytisch oder hydrierend gespalten werden.

Ausgangsstoffe der allgemeinen Formel II beziehungsweise III können beispielsweise wie folgt erhalten werden.
1. Durch Umsetzung einer Verbindung der Formel VII oder VIII 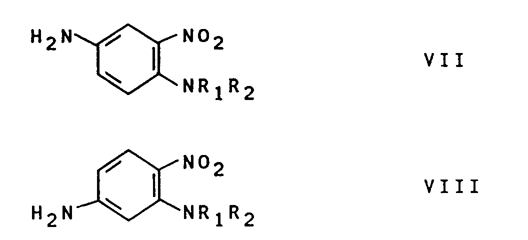 mit einem Aldehyd Ar-Alk'-CHO, wobei Alk die angegebenen Bedeutungen hat und Alk' eine gerade oder verzweigte entsprechende Alkylengruppe mit 0 - 8 C-Atomen ist, Reduktion der erhaltenen Schiff'schen Base in der hierfür üblichen Weise und gegebenenfalls Einführung der Reste R₁, R₂ und R₅.
2. Durch Acylierung von Verbindungen der Formel VII oder VIII mit einer Verbindung Ar-Alk-COHal, wobei Ar und Alk die angegebenen Bedeutungen haben und Hal Chlor, Brom oder Jod bedeuted und gegebenenfalls Einführung der Reste R₁, R₂ und R₅.
   Weiterhin können Ausgangsstoffe der Formel II, worin n = 0 ist (CO entfällt), erhalten werden durch Umsetzung einer Verbindung der Formel IX 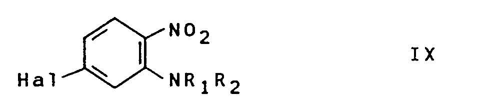 worin Hal Fluor, Chlor oder Brom bedeuted mit einem Amin der Formel Ar-Alk-NH₂, worin Ar und Alk die angegebenen Bedeutungen haben, zweckmäßig in Gegenwart eines basischen Stoffes (tertäres Amin) und gegebenenfalls anschließende Einführung der Reste R₁, R₂ und R₅.

Ausgangsstoffe der Formel III können beispielsweise auch wie folgt erhalten werden:
Eine Verbindung der Formel 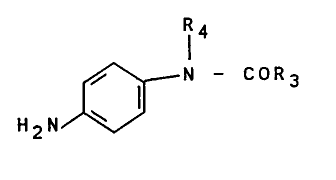
wird in üblicher Weise mit Phthalsäureanhydrid (beispielsweise in Eisessig bei 90 ^{o}C) umgesetzt, das erhaltene Reaktionsprodukt, bei dem die 4-ständige freie Aminogruppe nur durch den Phthalylrest geschützt ist, wird nun in bekannter Weise in 3-Stellung zur Phthalylgruppe nitriert (zum Beispiel mit rauchender Salpetersäure in Eisessig bei 90-100 ^{o}C). Anschließend wird der Phthalylrest in bekannter Weise (zum Beispiel mit Hydrazinhydrat) in einem inerten Mittel wie 1,2-Dimethoxyethan abgespalten und man erhält die Verbindung der Formel 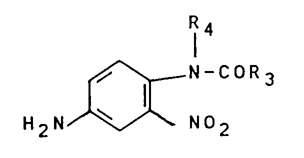
In dieser Verbindung können nun in die freie Aminogruppe nacheinander die Reste Ar-Alk-(CO)ₙ- und R₅ in hierfür üblicher Weise (zum Beispiel wie unter Verfahren b angegeben) eingeführt werden und gegebenenfalls der Rest -COR₃ in bekannter Weise abgespalten werden (Ersatz von -COR₃ durch Wasserstoff).

Die zuvor beschriebenen Umsetzungen zur Herstellung der Ausgangsstoffe werden in der üblichen Weise wie bei Beispiel 1 beschrieben ist beziehungsweise analog hierzu vorgenommen. Die Einführung der Reste R₁, R₂ und R₅ erfolgt ebenfalls in der hierfür üblichen Weise und wie in der Anmeldung (Beschreibung) angegeben ist.

### Zu dem Verfahren b) Umsetzung einer Verbindung der Formel IV mit einer Verbindung der Formel V oder VI

Das Verfahren b) wird zweckmäßig bei Temperaturen zwischen 0 bis 250 ^{o}C insbesondere 20 bis 140 ^{o}C ausgeführt.

Als Lösungsmittel oder Suspensionsmittel für die Verfahren kommen beispielsweise in Frage: Wasser, aliphatische Alkohole (Ethanol, Propanol, Butanol), aromatische Kohlenwasserstoffe (Benzol, Xyol, Toluol), niedere aliphatische Säureamide (Dimethylformamid), alicyclische und cyclische gesättigte Ether (Diäthyläther, Dioxan) N-Methylpyrrolidon, Dimethylsulfoxyd, Sulfolan, Tetramethylharnstoff und Gemische dieser Mittel.

Unter den Kondensationsmitteln, die für das Verfahren b) im Falle der Reaktionen einer Verbindung der Formel IV mit einer Verbindung der Formel V in Betracht kommen, sind in erster Linie beispielsweise Natriumacetat, Natriumamid, Alkalicarbonate und tertiäre Amine zu nennen. Gegebenenfalls können auch Zinkchlorid, Phosphoroxychlorid, p-Toluolsulfonsäure, Jod und dergleichen als Kondensationsmittel dienen.

Das Verfahren wird in Gegenwart von Wasserstoff durchgeführt, falls eine Verbindung der Formel VI zur Anwendung kommt. Als Katalysatoren kommen hierbei übliche Hydrierungskatalysatoren in Betracht, vorzugsweise metallische Hydrierungskatalysesatoren wie Raney-Nickel, Platin, Palladium. Es können aber auch Alkaliborhydride (NaBH₄) Lithiumborhydrid, Natriumcyanoborhydrid verwendet werden.

Ausgangsstoffe der Formel IV können beispielsweise durch übliche Reduktion der entsprechenden Nitro-Verbindungen (zum Beispiel der Formel VIII) erhalten werden. Diese Reduktion kann zum Beispiel so erfolgen wie bei dem Verfahren a) und in den Beispielen angegeben ist.
Ausgangsstoffe der Formel V beziehungsweise VI sind bekannt.
Die Einführung der Reste R₁, R₂, R₄ und R₅ einschließlich der Gruppe COR₃ gemäß dem Verfahren a) und/oder b) erfolgt in der hierfür üblichen Weise beispielsweise durch folgende Reaktionen:
Durch Alkylierung beziehungsweise Acylierung:
Insbesondere handelt es sich hier um die Acylierung oder Alkylierung von Aminogruppen.
Die Alkylierung beziehungsweise Acylierung erfolgt beispielsweise durch Umsetzung mit Verbindungen der Formel R-Hal, ArSO₂OR und SO₂(OR)₂, wobei Hal ein Halogenatom (insbesondere Chlor, Brom oder Jod) und Ar ein aromatischer Rest (zum Beispiel ein gegebenenfalls durch einen oder mehrere niedere Alkylreste substituierter Phenyl- oder Naphthylrest ist und R die Reste C₁-C₆-Alkyl Ar-C₁-C₆-Alkyl, Ar-C₁-C₆-Alkyl, wo die C₁-C₆-Alkylgruppe zusätzlich einen weiteren Rest Ar enthält oder Ar bedeutet. Beispiele sind p-Toluolsulfonsäure-C₁-C₆-alkylester,
C₁-C₆-Dialkylsulfate, C₁-C₆-Alkylhalogenide, und ähnliche. Bei den zuvor genanntgen Verbindungen kann die Arylgruppe jeweils entsprechend der Bedeutung von Ar substituiert sein. Die Alkylierungs- und Acylierungsreaktion wird gegebenenfalls unter Zusatz von üblichen säurebindenden Mitteln, wie Alkalihydroxiden, Alkalicarbonaten, Alkalihydrocarbonaten, Alkalihydrogencarbonaten, Erdalkalicarbonaten, Erdalkaliacetaten, tertiären Aminen (zum Beispiel Trialkylamin wie Triethylamin), Pyridin oder auch Alkalihydriden bei Temperaturen zwischen 0 und 200 ^{o}C, vorzugsweise 40 und 140 ^{o}C in inerten Lösungsmitteln oder Suspensionsmitteln durchgeführt. Als Lösungs-oder Dispergiermittel kommen beispielsweise in Betracht: aromatische Kohlenwasserstoffe wie zum Beispiel Benzol, Toluol Xylol; aliphatische Ketone wie zum Beispiel Aceton, Methylethylketon; halogenierte Kohlenwasserstoffe wie zum Beispiel Chloroform, Tetrachlorkohlenstoff, Chlorbenzol, Methylenchlorid; aliphatische Ether wie zum Beispiel Butylether; cyclische Ether wie zum Beispiel Tetrahydrofuran, Dioxan; Sulfoxyde wie zum Beispiel Dimethylsulfoxid; tertiäre Säureamide wie zum Beispiel Dimethylformamid, N-Methylpyrrolidon, Hexamethylphosphorsäuretriamid; aliphatische Alkohole wie Methanol, Ethanol, Isopropanol, Amylalkohol, tert.-Butanol, cycloaliphatische Kohlenwasserstoffe wie Cyclohexan. Auch wässrige Mischungen der genannten Lösungsmittel können verwendet werden. Häufig arbeitet man bei der Rückflußtemperatur der verwendeten Lösungs- beziehungsweise Dispergiermittel. Häufig werden die Alkylierungsreaktionskomponenten im Überschuß eingesetzt. Die Alkylierung kann auch in Gegenwart von Tetraalkylammoniumsalzen (insbesondere der Halogenide) in Kombination mit Alkalihydroxiden bei Temperaturen zwischen 0 -100 ^{o}C, vorzugsweise 20 - 80 ^{o}C, in einem aprotischen Lösungsmittel oder auch in Chloroform oder Methylenchlorid vorgenommen werden. Als aprotische Lösungsmittel kommen insbesondere in Betracht: tertiäre Amide (Dimethylformamid, N-Methyl-Pyrrolidon, Hexamethylphosphorsäuretriamid) Dimethylsulfoxid, Acetonitril, Dimethoxyethan, Aceton, Tetrahydrofuran.

Bei der Acylierung werden zum Beispiel in Aminogruppen die Gruppe -COR₃, die Gruppe -CO-C₁-C₆-Alkyl, -CO-C₂-C₆-Alkenyl, -CO-C₂-C₆-Alkinyl, die C₁-C₆-Alkoxycarbonylgruppe oder die folgenden Gruppen eingeführt:
-COAr, -CO-OAr, --CONH₂
-CONH-C₁-C₆-Alkyl,-CON(C₁-C₆-Alkyl)₂, -CONHAr,
-SO₂-C₁-C₆-Alkyl, -SO₂Ar, -SO₂-Nitrophenyl.
Man verfährt hierbei in an sich bekannter Weise beispielsweise verwendet man die entsprechenden Säurehalogenide (chloride, bromide) wie zum Beispiel Carb-C₁-C₆-alkoxyhalogenide oder entsprechende Anhydride). Die Reaktionstemperaturen liegen vorzugsweise zwischen 20 und 120 ^{o}C.

Gegebenenfalls kann man bei der Alkylierung und der Acylierung auch so vorgehen, daß man zuerst von der zu alkylierenden beziehungsweise acylierenden Verbindung eine Alkaliverbindung (Natrium-, Kalium- oder auch Lithiumsalz zum Beispiel) herstellt, indem man sie in einem inerten Lösungsmittel wie Dioxan, Dimethylformamid, Benzol oder Toluol mit einem Alkalimetall, Alkalihydrid oder Alkaliamiden (insbesondere Natrium oder Natriumverbindungen) oder Butyllithium bei Temperaturen zwischen 0 und 150 ^{o}C umsetzt und dann das alkylierende Agens zufügt.

Anstelle der angeführten Alkylierungs- und Acylierungsmittel können auch andere in der Chemie gebräuchliche chemisch-äquivalente Mittel verwendet werden (siehe zum Beispiel auch L.F. und Mary Fieser "Reagents for Organic Synthesis", John Wiley and Sons, Inc., New York, 1967, Vol. 1, Seiten 1303-4 und Vol.2, Seite 471.

Vorhandene Acylgruppen wie zum Beispiel Carb-C₁-C₆-alkoxygruppen, C₂-C₆-Alkanoylgruppen und ähnliche Gruppen können solvolytisch abgespalten werden. Diese Abspaltung erfolgt in bekannter Weise beispielsweise durch Verseifung mit Säuren (Mineralsäuren wie Salzsäure, Schwefelsäure, insbesondere konzentrierten Halogenwasserstoffsäuren wie HBr/Eisessig) oder mittels basischer Substanzen (Pottasche, Soda, wässrige Alkalilösungen, alkoholische Alkalilösungen, wässriges NH₃) bei Temperaturen zwischen 10 und 150 ^{o}C, insbesondere 20 - 100 ^{o}C.

Die erfindungsgemäßen Verbindungen zeigen beispielsweise beim Maximalen Elektrokrampf-Test (MES) eine gute antiepileptische Wirkung.
Beispielsweise wird bei obengenannter Versuchsmethode bei einer Dosis von 6 mg/kg Körpergewicht Ratte eine 50%ige Hemmung der durch elektrische Stimulation hervorgerufenen Krämpfe erzielt.

Die niedrigste bereits wirksame Dosis in dem obenangegebenen Tierversuch ist beispielsweise
2 mg/kg oral
0,5 mg/kg intravenös.

Als allgemeiner Dosisbereich für die Wirkung (Tierversuch wie oben) kommt beispielsweise in Frage:
2 - 20 mg/kg oral, insbesondere 10 mg/kg
0,5 - 5 mg/kg intravenös, insbesondere 2 mg/kg.

Die Wirkungsrichtung der erfindungsgemäßen Verbindungen ist mit der Wirkung des bekannten Arzneimittelwirkstoffes Diazepam vergleichbar, jedoch weisen die erfindungsgemäßen Verbindungen eine größere therapeutische Breite auf.

Die erfindungsgemäßen Verbindungen stellen insbesondere wirkungsvolle Antiepileptika dar.

Die pharmazeutichen Zubereitungen enthalten im allgemeinen zwischen 10 bis 100, vorzugsweise 30 bis 60 mg der erfindungsgemäßen aktiven Komponente(n).

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 30 und 60 mg oder Lösungen, die zwischen 0,1 bis 5 Gewichtsprozent an aktiver Substanz enthalten.

Die Einzeldosis der erfindungsgemäßen aktiven Komponenten kann beispielsweise liegen
a) bei oralen Arzneiformen zwischen 20 und 80 mg vorzugsweise 30 - 60 mg
b) bei parenteralen Arzneiformen (zum Beispiel intravenös, intramuskulär zwischen 5 - 20 mg, vorzugsweise 8 - 16 mg.
   -(Die Dosen sind jeweils bezogen auf die freie Base)-
Beispielsweise können 3mal täglich 1 bis 3 Tabletten mit einem Gehalt von 30 - 60 mg wirksamer Substanz oder zum Beispiel bei intravenöser Injektion 1 bis 3mal täglich eine Ampulle von 3 bis 5 ml Inhalt mit 8 bis 16 mg Substanz empfohlen werden. Bei oraler Verabreichung ist die minimale tägliche Dosis beispielsweise 90 mg; die maximale tägliche Dosis bei oraler Verabreichung soll nicht über 270 mg liegen.

Für die Behandlung von Hunden und Katzen liegt die orale Einzeldosis im allgemeinen zwischen ungefähr 2 und 20 mg/kg Körpergewicht: die parenterale Dosis ungefähr zwischen 1 und 5 mg/kg Körpergewicht.

Die Arzneimittel können in der Humanmedizin, der Veterinärmedizin sowie in der Landwirtschaft allein oder im Gemisch mit anderen pharmakologisch aktiven Stoffen verwendet werden.

### Beispiele:

### Beispiel 1

### 2-Amino-4-(4-fluorbenzylamino)-1-ethoxycarbonylaminobenzol.

### Variante A

Eine Suspension von 5,2 g (20 mmol) 2-Amino-5-(4-fluorbenzylamino)-nitrobenzol oder 5,2 g (20 mmol) 2-Amino-4-(4-fluorbenzyl-amino)-nitrobenzol in 100 ml Dioxan wird bei 55 - 60 ^{o}C unter Normaldruck in Gegenwart von 2 g Raney-Nickel hydriert. Nach beendeter Wasserstoffaufnahme filtriert man den Katalysator unter Schutzgas ab und versetzt das Filtrat mit 3,2 g (25 mmol) Di-isopropylethylamin. Zu der erhaltenen Lösung tropft man unter Rühren und Schutzgasatmosphäre (zum Beispiel Stickstoff) bei 18 - 20 ^{o}C eine Lösung von 2,3 g (21 mmol) Chlorameisensäureethylester in 15 ml Dioxan zu. Nach beendeter Zugabe rührt man 1 Stunde bei Raumtemperatur nach und tropft anschließend unter Rühren und Eiskühlung 10 ml 6N ethanolische Salzsäure zu. Dabei kristallisiert allmählich ein farbloser Feststoff aus. Man rührt 2 Stunden bei Raumtemperatur nach und saugt das ausgefallene Produkt ab. Nach Umkristallisation aus Ethanol/Ether erhält man 5,5 g (73 % der Theorie) 2-Amino-4-(4-fluorbenzylamino)-1-ethoxycarbonylamino-benzol als Dihydrochlorid in Form farbloser bis leicht rosa gefärbter Kristalle.
F. des Dihydrochlorids 182 - 186 ^{o}C.

### Herstellung der Ausgangssubstanzen.

### 2-Amino-5-(4-fluorbenzylamino)-nitrobenzol

Eine Lösung aus 25,9 g (100 mmol) 2-Amino-5-(4-fluorbenzylidenamino)-nitrobenzol in 250 ml Dioxan/Methanol-Gemisch (4:1) wird unter Rühren portionsweise mit 5,7 g (150 mmol) Natriumtetrahydridoboranat versetzt. Nach beendeter Zugabe rührt man 2 Stunden bei Raumtemperatur nach und engt die Reaktionslösung daraufhin im Vakuum bis zur Trockene ein. Der verbleibende dunkelrote Rückstand wird in 100 ml Wasser aufgenommen und die resultierende Suspension viermal mit je 200 ml Dichlormethan extrahiert. Die vereinigten organischen Phasen werden mit Natriumsulfat getrocknet und anschließend im Vakuum eingeengt. Das zurückbleibende dunkelrote Öl kristallisiert man aus 100 ml Toluol. Nach Trocknen im Vakuum erhält man 21,0 g (80 % der Theorie) 2-Amino-5-(4-fluorbenzylamino)-nitrobenzol als dunkelrote Kristalle.
F.: 111 - 112 ^{o}C.

### 2-Amino-5-(4-fluorbenzylidenamino)-nitrobenzol kann beispielsweise wie folgt erhalten werden:

Eine Lösung aus 15,3 g (100 mmol) 2-Nitro-p-phenylendiamin und 13,6 g (110 mmol) 4-Fluorbenzaldehyd in 400 ml Xylol wird in Gegenwart von 0,5 g eines sauren Ionenaustauschers (zum Beispiel Nafion) 3 Stunden am Wasserabscheider unter Rückfluß erhitzt. Man filtriert heiß von unlöslichen Bestandteilen ab und läßt über Nacht bei Raumtemperatur stehen. Dabei kristallisiert ein orangegelber Feststoff aus. Dieser wird abgesaugt, mit wenig Toluol nachgewaschen und im Vakuum getrocknet. Man erhält 22,0 g (85 % der Theorie) 2-Amino-5-(4-fluorbenzylidenamino)-nitrobenzol als orangegelbe Kristalle.
F.: 136 - 139 ^{o}C.

### 2-Amino-4-(4-fluorbenzylamino)-nitrobenzol

Eine Lösung aus 15,6 g (100 mmol) 2-Amino-4-fluor-nitrobenzol, 22,5 g (180 mmol) 4-Fluorbenzylamin und 20,2 g (200 mmol) Triethylamin in 150 ml Dimethylsulfoxid wird unter Rühren und Schutzgasatmosphäre 30 Stunden auf 60 - 70 ^{o}C erwärmt. Nach beendeter Reaktionszeit destilliert man das Lösungsmittel im Vakuum ab, nimmt den Rückstand in 500 ml 0,5 N Salzsäure auf und rührt 30 Minuten intensiv bei Raumtemperatur. Der ausgefallene Feststoff wird abgesaugt und einer Heißdampfextraktion mit Toluol unterworfen. Das nach zwölfstündigem Stehenlassen bei 0 - 4 ^{o}C aus der Toluolphase kristallisierende Produkt wird abgesaugt und im Vakuum getrocknet. Man erhält 22,2 g (85 % der Theorie). Aus der Toluolphase kristallisieren nach 12-stündigem Stehen bei 0 - 4 ^{o}C 2-Amino-4-(4-fluorbenzylamino)-nitrobenzol als orangegelbe Kristalle.
F.: 180 ^{o}C.

### Variante B

Eine Suspension von 13,3 g (40 mmol) 2-Ethoxycarbonylamino-5-(4-fluorbenzylamino)-nitrobenzol in 150 ml 1,2-Dimethoxyethan wird bei 55 - 60 ^{o}C unter Normaldruck in Gegenwart von 2 g Raney-Nickel hydriert. Nach beendeter Wasserstoffaufnahme filtriert man den Katalysator unter Schutzgas ab und engt das Filtrat im Vakuum ein. Den verbleibenden Rückstand nimmt man in 150 ml Ethanol auf und tropft zu der erhaltenen Lösung unter Rühren und Schutzgasatmosphäre 20 ml 6 N ethanolische Salzsäure zu. Nach beendeter Zugabe rührt man 1 Stunde nach, saugt den ausgefallenen Feststoff ab und wäscht mit Ethanol nach. Nach Trocknen im Vakuum erhält man 13,5 g (89 % der Theorie) der genannten Verbindung als farblose bis leicht rosa gefärbte Kristalle.
F.: 186 - 189 ^{o}C.

### Herstellung der Ausgangssubstanz

2-Ethoxycarbonylamino-5-(4-fluorbenzylamino)-nitrobenzol 39,8 g (120 mmol) 2-Ethoxycarbonylamino-5-(4-fluorbenzylidenamino)-nitrobenzol (hergestellt aus 33,8 g (150 mmol) 5-Amino-2-ethoxycarbonylamino-nitrobenzol und 19,2 g (155 mmol) 4-Fluorbenzaldehyd) werden wie zuvor angegeben mit 3,8 g (100 mmol) Natriumtetrahydridoboranat umgesetzt. Nach entsprechender Aufarbeitung erhält man 31,3 g (78 % der Theorie) 2-Ethoxycarbonylamino-5-(4-fluorbenzylamino)-nitrobenzol als dunkelrote Kristalle.
F.: 85 - 87 ^{o}C.

### Das 5-Amino-2-ethoxycarbonylamino-nitro-benzol kann zum Beispiel wie folgt erhalten werden:

Zu einer Suspension von 142,1 g (0,4 mol) 2-Ethoxycarbonylamino-5-phthalimido-nitrobenzol in 700 ml 1,2-Dimethoxyethan tropft man unter Rühren in der Siedehitze 21,0 g (0,42 mol) Hydrazinhydrat zu und läßt 2 Stunden nachreagieren. Nach Abkühlen auf Raumtemperatur filtriert man von ausgefallenem Phthalhydrazid ab und engt das dunkelrote Filtrat im Vakuum bis zur Trockene ein. Der verbleibende feste Rückstand wird aus ca. 150 ml Toluol umkristallisiert. Man erhält 84,4 g 5-Amino-2-ethoxycarbonylaminonitrobenzol als dunkelrote Kristalle.
F.: 105 - 107 ^{o}C.
Das 2-Ethoxycarbonylamino-5-phthalimido-nitrobenzol erhält man zum Beispiel wie folgt:
Zu einer Lösung von 90,1 g (0,5 mol) N-Ethoxycarbonyl-p-phenylendiamin in 1,5 l Eisessig gibt man unter Rühren und Schutzgasatmosphäre bei 90 ^{o}C portionsweise 75,5 g (0,51 mol) Phthalsäureanhydrid. Nach beendeter Zugabe läßt man 60 Minuten nachreagieren. Zu der resultierenden Suspension tropft man daraufhin 27,5 ml rauchende Salpetersäure zu und läßt nach beendeter Zugabe 2 Stunden bei 100 ^{o}C nachreagieren. Nach Abkühlen auf Raumtemperatur wird der ausgefallene Feststoff abfiltriert und mehrmals mit insgesamt 2,5 l Wasser neutral gewaschen. Nach Trocknen im Vakuum erhält man 149,9 g der Phthalimido-Verbindung als gelbe Kristalle.
F.: 215 - 216 ^{o}C.

### Beispiel 2

### 2-Amino-4-(4-trifluormethylbenzylamino)-1-ethoxycarbonylamino-benzol

### Variante A

Eine Suspension aus 9,3 g (30 mmol) 2-Amino-5-(4-trifluormethylbenzylamino)-nitrobenzol wird analog Beispiel 1 (Variante A) hydriert und anschließend mit 3,4 g (31 mmol) Chlorameisensäureethylester umgesetzt. Man erhält 5,8 g (45 % der Theorie) des genannten Endproduktes als Dihydrochlorid in Form farbloser bis schwach rosa gefärbter Kristalle.
F.: 159 - 162 ^{o}C.

### Variante B

Eine Suspension aus 65,2 g (170 mmol) 2-Ethoxycarbonylamino-5-(4-trifluormethylbenzylamino)-nitrobenzol wird analog Beispiel 1 (Variante B) hydriert und entsprechend aufgearbeitet. Man erhält 52,2 g (72 % der Theorie) des genannten Endproduktes als Dihydrochlorid in Form farbloser Kristalle.
F.: 160 - 162 ^{o}C.

Die entsprechenden Ausgangssubstanzen werden, wie bei Beispiel 1 beschrieben ist, in analoger Weise hergestellt.

### Beispiel 3

### 2-Amino-4-benzylamino-1-ethoxycarbonylamino-benzol.

Eine Suspension aus 12,6 g (40 mmol 5-Benzylamino-2-ethoxycarbonyl-amino-nitrobenzol wird analog Beispiel 1 (Variante B) hydriert und entsprechend aufgearbeitet. Man erhält 52,2 g (72 % der Theorie) Endprodukt als Dihydrochlorid: farblose bis schwach rosa gefärbte Kristalle.
F.: 178 - 181 ^{o}C.

### Beispiel 4

### 2-Amino-4-(3,5-dichlorbenzylamino)-1-ethoxycarbonylamino-benzol

Eine Suspension aus 15,5 g (50 mmol) 2-Amino-5-(3,5-dichlorbenzylamino)-nitrobenzol wird analog Beispiel 1 (Variante A) hydriert und entsprechend mit 5,5 g (51 mmol) Chlorameisensäureethylester umgesetzt. Man erhält 10,8 g (51 % der Theorie) Endprodukt als Dihydrochlorid: farblose bis schwach rosa gefärbte Kirstalle.
F.: 134 - 136 ^{o}C.

### Beispiel 5

### 2-Amino-4-(3,5-dichlorbenzylamino)-1-propyloxycarbonylamino-benzol

Eine Suspension aus 15,5 g (50 mmol 2-Amino-5-(3,5-dichlorbenzylamino)-nitrobenzol wird analog Beispiel 1 (Variante A) hydriert und entsprechend mit 6,2 g (51 mmol) Chlorameisensäurepropylester umgesetzt. Man erhält 10,8 g Endprodukt in Form des Dihydrochlorids. Dieses bildet farblose bis schwach rosa gefärbte Kristalle.
F.: 154 - 155 ^{o}C.

### Beispiel 6

### 2-Amino-4-(2-chlorbenzylamino)-1-(diethylcarbamoylamino)-benzol

Eine Suspension aus 8,3 g (30 mmol) 2-Amino-5-(2-chlorbenzylamino)-nitrobenzol wird analog Beispiel 1 (Variante A) hydriert und entsprechend mit 4,2 g (31 mmol) N,N-Dimethylcarbamoylchlorid umgesetzt. Man erhält 6,5 g Endprodukt in Form des Dihydrochlorids. Dieses bildet farblose bis schwach rosa gefärbte Kristalle.
F.: 153 - 155 ^{o}C.

### Beispiel 7

### 2-Amino-4-(2,4-dichlorbenzylamino)-1-(dimethylcarbamoylamino)-benzol

Eine Suspension aus 31,2 g (100 mmol) 2-Amino-5-(2,4dichlorbenzyl-amino)-nitrobenzol wird analog Beispiel 1 (Variante A) hydriert und entsprechend mit 13,9 g (101 mmol) N,N-Dimethylcarbamoylchlorid umgesetzt. Man erhält 12,4 g Endprodukt in Form des Dihydrochlorids. Dieses bildet farblose bis schwach rosa gefärbte Kristalle.
F.: 147 - 152 ^{o}C.

### Beispiel 8

### 1,2-Diacetylamino-4-(4-fluorbenzylamino)-benzol

9,4 g (30 mmol) 4-(4-Fluorbenzylidenamino)-N,N'-diacetyl-o-phenylendiamin werden in 250 ml Dioxan/Methanol-Gemisch (4:1) wie unter Beispiel 1 beschrieben ist mit 0,9 g (25 mmol) Natriumtetrahydriodoboranat reduziert. Die erhaltene freie Base wird anschließend direkt mit alkoholischer Salzsäure in das Hydrochlorid überführt. Man erhält 7,2 g Endprodukt in Form des Monohydrochlorids. Dieses bildet farblose Kristalle.
F.: 165 - 168 ^{o}C.

Das 4-(4-Fluorbenzylidenamino)-N,N'-diacetyl-o-phenylendiamin wird beispielsweise durch Umsetzung von 8,3 g (40 mmol) 4-Amino-N,N'-diacetyl-o-phenylendiamin mit 5,1 g (41 mmol) 4-Fluorbenzaldehyd erhalten (analog wie unter Beispiel 1 beschrieben). Ausbeute 11,0 g gelbe Kristalle.
F.: 152 - 156 ^{o}C.

## Patentansprüche

1. 1,2,4-Triaminobenzol-Derivate der allgemeinen Formel 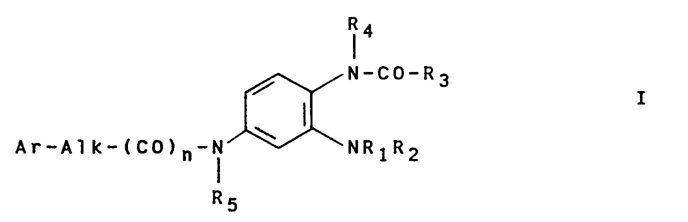 worin die Symbole R₁, R₂, R₃, R₄, R₅, Ar und Alk die folgenden Bedeutungen haben:
R₁: Wasserstoff, C₁-C₆-Alkyl, C₂-C₆-Alkanoyl oder der Rest Ar;
R₂: Wasserstoff oder C₁-C₆-Alkyl;
R₃: C₁-C₆-Alkoxy, NH₂, C₁-C₆-Alkylamino, C₁-C₆-Dialkylamino, Amino, welches durch den Rest Ar substituiert ist, C₁-C₆-Alkyl, C₂-C₆-Alkenyl, C₂-C₆-Alkinyl, der Rest Ar oder der Rest Ar0-;
R₄: Wasserstoff, C₁-C₆-Alkyl oder der Rest Ar;
R₅: Wasserstoff, C₁-C₆-Alkyl oder der Rest Ar;
Alk: Eine gerade oder verzweigte Alkylengruppe mit 1 - 9 C-Atomen, die auch durch den Rest Ar substituiert sein kann;
Ar: Phenylrest, der durch die Reste R₆, R₇ und/oder R₈ substituiert ist, wobei diese Reste R₆, R₇ und R₈ gleich oder verschieden sind und C₁-C₆-Alkyl, C₃-C₇-Cycloalkyl, Hydroxy, C₁-C₆-Alkoxy, C₂-C₆-Alkanoyloxy, Halogen, Hydroxy, C₁-C₆-Halogenalkyl, -CN, -NH₂, -NH-C₁-C₆-Alkyl, -N(C₁-C₆-Alkyl)₂, -CO₂H, -CO-C₁-C₆-Alkyl, -CO-O-C₁-C₆-Alkyl, -COAr, -CO-OAr, -CONH₂, -CONH-C₁-C₆-Alkyl, -CON(C₁-C₆-Alkyl)₂, -CONHAr, -NH-CO-C₁-C₆-Alkyl, -NHCO-Ar, NHCO-C₁-C₆-Alkoxy, -NH-CO-OAr, -NHCO-NH₂, -NHCO-N(C₁-C₆-Alkyl)₂, -NHCO-NHAr, -NH-SO₂-C₁-C₆-Alkyl, -NH-SO₂Ar, -NH-SO₂-Nitrophenyl, -SO₂-OH, -SO₂-C₁-C₆-Alkyl, -SO₂-Ar, -SO₂-C₁-C₆-Alkoxy, -SO₂-OAr, -SO₂-NH₂, -SO₂-NH-C₁-C₆-Alkyl, -SO₂-N(C₁-C₆-Alkyl)₂, -SO₂-NHAr, -SO₂-C₁-C₆-Alkoxy bedeuten;
n: 0 oder 1;
und deren pharmazeutisch verträgliche Säureadditionssalze, ausgenommen die Verbindung 2-Carbethoxy-amino-5-(2,4,6-trimethylbenzylamino)-anilin und deren Hydrochlorid.

2. Verfahren zur Herstellung von 1,2,4-Triaminobenzol-Derivaten der allgemeinen Formel 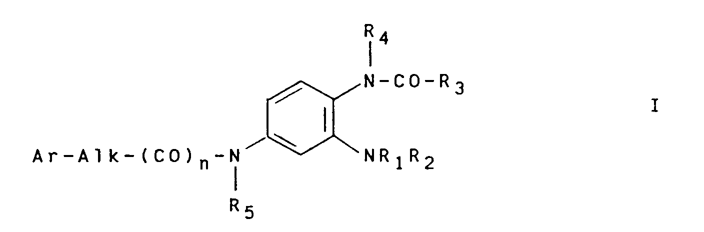 worin die Symbole R₁, R₂, R₃, R₄, R₅, Ar und Alk die folgenden Bedeutungen haben:
R₁: Wasserstoff, C₁-C₆-Alkyl, C₂-C₆-Alkanoyl oder der Rest Ar;
R₂: Wasserstoff oder C₁-C₆-Alkyl;
R₃: C₁-C₆-Alkoxy, NH₂, C₁-C₆-Alkylamino, C₁-C₆-Dialkylamino, Amino, welches durch den Rest Ar substituiert ist, C₁-C₆-Alkyl, C₂-C₆-Alkenyl, C₂-C₆-Alkinyl, der Rest Ar oder der Rest Ar0-;
R₄: Wasserstoff, C₁-C₆-Alkyl oder der Rest Ar;
R₅: Wasserstoff, C₁-C₆-Alkyl oder der Rest Ar;
Alk: Eine gerade oder verzweigte Alkylengruppe mit 1 - 9 C-Atomen, die auch durch den Rest Ar substituiert sein kann;
Ar: Phenylrest, der durch die Reste R₆, R₇ und/oder R₈ substituiert ist, wobei diese Reste R₆, R₇ und R₈ gleich oder verschieden sind und C₁-C₆-Alkyl, C₃-C₇-Cycloalkyl, Hydroxy, C₁-C₆-Alkoxy, C₂-C₆-Alkanoyloxy, Halogen, Hydroxy, C₁-C₆-Halogenalkyl, -CN, -NH₂, -NH-C₁-C₆-Alkyl, -N(C₁-C₆-Alkyl)₂, -CO₂H, -CO-C₁-C₆-Alkyl, -CO-O-C₁-C₆-Alkyl, -COAr, -CO-OAr, -CONH₂, -CONH-C₁-C₆-Alkyl, -CON(C₁-C₆-Alkyl)₂, -CONHAr, -NH-CO-C₁-C₆-Alkyl, -NHCO-Ar, NHCO-C₁-C₆-Alkoxy, -NH-CO-OAr, -NHCO-NH₂, -NHCO-N(C₁-C₆-Alkyl)₂, -NHCO-NHAr, -NH-SO₂-C₁-C₆-Alkyl, -NH-SO₂Ar, -NH-SO₂-Nitrophenyl, -SO₂-OH, -SO₂-C₁-C₆-Alkyl, -SO₂-Ar, -SO₂-C₁-C₆-Alkoxy, -SO₂-OAr, -SO₂-NH₂, -SO₂-NH-C₁-C₆-Alkyl, -SO₂-N(C₁-C₆-Alkyl)₂, -SO₂-NHAr, -SO₂-C₁-C₆-Alkoxy bedeuten;
n: 0 oder 1;
und deren pharmazeutisch verträgliche Säureadditionssalze, ausgenommen die Verbindung 2-Carbethoxy-amino-5-(2,4,6-trimethylbenzylamino)-anilin und deren Hydrochlorid,
dadurch gekennzeichnet,
daß man in an sich bekannter Weise
a) in einer Verbindung der Formel 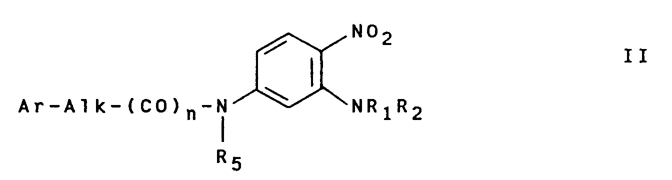 worin R₁, R₂, R₅, Alk, Ar und n die angegebenen Bedeutungen haben oder in einer Verbindung der Formel 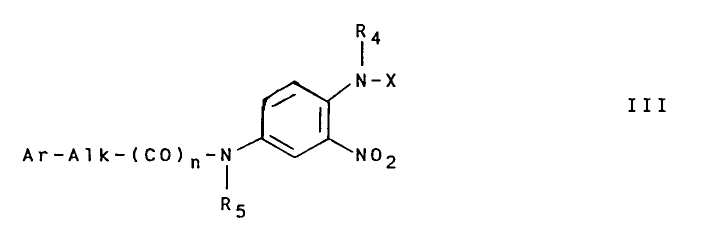 worin X Wasserstoff oder die Gruppe -COR₃ ist und R₃, R₄, R₅, Alk, Ar und n die angegebenen Bedeutungen haben, die Nitrogruppe zur Aminogruppe reduziert, durch Acylierung die Gruppe -COR₃ einführt, falls diese in den Ausgangsstoffen II oder III nicht vorhanden ist und gegebenenfalls nach oder vor Einführung der Gruppe -COR₃ durch Alkylierung, Arylierung und/oder Acylierung die Reste R₁, R₂, R₄ und R₅ einführt oder
b) eine Verbindung der Formel 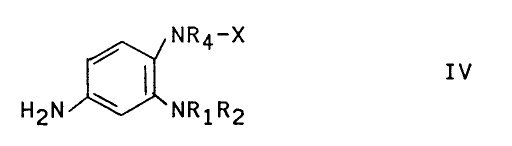 worin X Wasserstoff oder die Gruppe -COR₃ ist und R₁, R₂, R₃ und R₄ die angegebenen Bedeutungen haben mit einer Verbindung der Formel
Ar-Alk-(CO)ₙHal V
worin Ar, -Alk und n die angegebenen Bedeutungen haben und Hal Chlor, Brom oder Jod ist oder einer Verbindung der Formel
Ar-Alk'-CHO VI
worin Ar die angegebenen Bedeutungen hat und Alk' eine gerade oder verzweigte Alkylengruppe mit 0 - 8 C-Atomen ist,
umsetzt, wobei im letzteren Falle die Doppelbindung der erhaltenen Schiffschen Base zur Einfachbindung reduziert wird, gegebenenfalls durch Alkylierung, Arylierung und/oder Acylierung die Reste -COR₃, R₁, R₂, R₄ sowie R₅ einführt, gegebenenfalls in den nach den Verfahren a) oder b) erhaltenen Verbindungen den Rest R₃ gegen einen anderen Rest im Rahmen der für R₃ angegebenen Definition austauscht und die so erhaltenen Verbindungen in Säureadditionssalze überführt.

3. Arzneimittel enthaltend als Wirkstoff mindestens ein 1,2,4-Triaminobenzol-Derivat der allgemeinen Formel 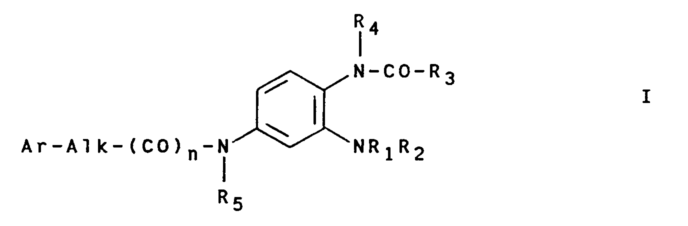 worin die Symbole R₁, R₂, R₃, R₄, R₅, Ar und Alk die folgenden Bedeutungen haben:
R₁: Wasserstoff, C₁-C₆-Alkyl, C₂-C₆-Alkanoyl oder der Rest Ar;
R₂: Wasserstoff oder C₁-C₆-Alkyl;
R₃: C₁-C₆-Alkoxy, NH₂, C₁-C₆-Alkylamino, C₁-C₆-Dialkylamino, Amino, welches durch den Rest Ar substituiert ist, C₁-C₆-Alkyl, C₂-C₆-Alkenyl, C₂-C₆-Alkinyl, der Rest Ar oder der Rest Ar0-;
R₄: Wasserstoff, C₁-C₆-Alkyl oder der Rest Ar;
R₅: Wasserstoff, C₁-C₆-Alkyl oder der Rest Ar;
Alk: Eine gerade oder verzweigte Alkylengruppe mit 1 - 9 C-Atomen, die auch durch den Rest Ar substituiert sein kann;
Ar: Phenylrest, der durch die Reste R₆, R₇ und/oder R₈ substituiert ist, wobei diese Reste R₆, R₇ und R₈ gleich oder verschieden sind und C₁-C₆-Alkyl, C₃-C₇-Cycloalkyl, Hydroxy, C₁-C₆-Alkoxy, C₂-C₆-Alkanoyloxy, Halogen, Hydroxy, C₁-C₆-Halogenalkyl, -CN, -NH₂, -NH-C₁-C₆-Alkyl, -N(C₁-C₆-Alkyl)₂, -CO₂H, -CO-C₁-C₆-Alkyl, -CO-O-C₁-C₆-Alkyl, -COAr, -CO-OAr, -CONH₂, -CONH-C₁-C₆-Alkyl, -CON(C₁-C₆-Alkyl)₂, -CONHAr, -NH-CO-C₁-C₆-Alkyl, -NHCO-Ar, NHCO-C₁-C₆-Alkoxy, -NH-CO-OAr, -NHCO-NH₂, -NHCO-N(C₁-C₆-Alkyl)₂, -NHCO-NHAr, -NH-SO₂-C₁-C₆-Alkyl, -NH-SO₂Ar, -NH-SO₂-Nitrophenyl, -SO₂-OH, -SO₂-C₁-C₆-Alkyl, -SO₂-Ar, -SO₂-C₁-C₆-Alkoxy, -SO₂-OAr, -SO₂-NH₂, -SO₂-NH-C₁-C₆-Alkyl, -SO₂-N(C₁-C₆-Alkyl)₂, -SO₂-NHAr, -SO₂-C₁-C₆-Alkoxy bedeuten;
n: 0 oder 1;
oder dessen pharmazeutisch verträgliche Säureadditionssalze
sowie gegebenenfalls weitere übliche Hilfs- und Trägerstoffe beziehungsweise Verdünnungsmittel.

4. Verfahren zur Herstellung eines Arzneimittels, dadurch gekennzeichnet, daß mindestens eine Verbindung gemäß Formel I unter Einschluß der Verbindung 2-Carbethoxy-amino-5-(2,4,6-trimethylbenzylamino)-anilin und deren Hydrochlorid mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht wird.

5. Verwendung von Verbindungen der Formel 1 gemäß Anspruch 1 unter Einschluß der Verbindung 2-Carbethoxy-amino-5-(2,4,6-trimethylbenzylamino)-anilin und deren Hydrochlorid zur Herstellung von Arzneimitteln.

## Claims

1. 1,2,4-Triaminobenzene derivatives of the general formula 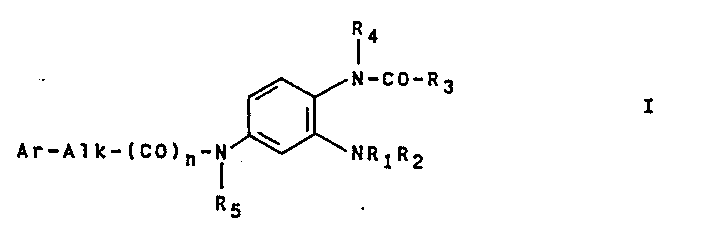 in which the symbols R₁, R₂, R₃, R₄, R₅, Ar and Alk have the following meanings:
R₁: hydrogen, C₁-C₆ alkyl, C₂-C₆ alkanoyl or the residue Ar;
R₂: hydrogen or C₁-C₆ alkyl;
R₃: C₁-C₆ alkoxy, NH₂, C₁-C₆ alkylamino, C₁-C₆ dialkylamino, amino which is substituted by the residue Ar, C₁-C₆ alkyl, C₂-C₆ alkenyl, C₂-C₆ alkynyl, the residue Ar or the residue ArO-;
R₄: hydrogen, C₁-C₆ alkyl or the residue Ar;
R₅: hydrogen, C₁-C₆ alkyl or the residue Ar;
Alk: a linear or branched alkylene group with 1-9 C atoms, which may also be substituted by the residue Ar;
Ar: phenyl residue which is substituted by the residues R₆, R₇ and/or R₈, wherein these residues R₆, R₇ and R₈ are identical or different and C₁-C₆ alkyl, C₃-C₇ cycloalkyl, hydroxy, C₁-C₆ alkoxy, C₂-C₆ alkanoyloxy, halogen, hydroxy, C₁-C₆ halogenoalkyl, -CN, -NH₂, -NH-C₁-C₆ alkyl, -N(C₁-C₆ alkyl)₂, -CO₂H, -CO-C₁-C₆ alkyl, -CO-O-C₁-C₆ alkyl, -COAr, -CO-OAr, -CONH₂, -CONH-C₁-C₆ alkyl, -CON(C₁-C₆ alkyl)₂, -CONHAr, -NH-CO-C₁-C₆ alkyl, -NHCO-Ar, NCHO-C₁-C₆ alkoxy, -NH-CO-OAr, -NHCO-NH₂, -NHCO-N(C₁-C₆ alkyl)₂, -NHCO-NHAr, -NH-SO₂-C₁-C₆ alkyl, -NH-SO₂Ar, -NH-SO₂-nitrophenyl, -SO₂-OH, -SO₂-C₁-C₆ alkyl, -SO₂-Ar, -SO₂-C₁-C₆ alkoxy, -SO₂-OAr, -SO₂-NH₂, -SO₂-NH-C₁-C₆ alkyl, -SO₂-N(C₁-C₆ alkyl)₂, -SO₂-NHAr, -SO₂-C₁-C₆ alkoxy;
n: 0 or 1;
and the pharmaceutically compatible acid addition salts thereof, with the exception of the compound 2-carbethoxy-amino-5-(2,4,6-trimethylbenzylamino)-aniline and the hydrochloride thereof.

2. Process for the production of 1,2,4-triaminobenzene derivatives of the general formula 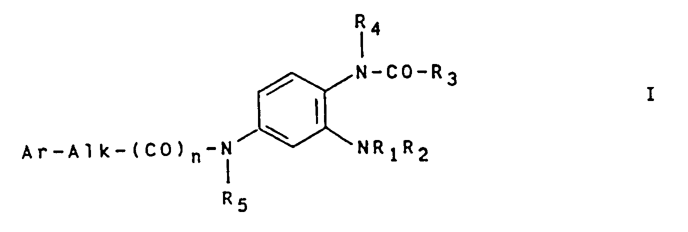 in which the symbols R₁, R₂, R₃, R₄, R₅, Ar and Alk have the following meanings:
R₁: hydrogen, C₁-C₆ alkyl, C₂-C₆ alkanoyl or the residue Ar;
R₂: hydrogen or C₁-C₆ alkyl;
R₃: C₁-C₆ alkoxy, NH₂, C₁-C₆ alkylamino, C₁-C₆ dialkylamino, amino which is substituted by the residue Ar, C₁-C₆ alkyl, C₂-C₆ alkenyl, C₂-C₆ alkynyl, the residue Ar or the residue ArO-;
R₄: hydrogen, C₁-C₆ alkyl or the residue Ar;
R₅: hydrogen, C₁-C₆ alkyl or the residue Ar;
Alk: a linear or branched alkylene group with 1-9 C atoms, which may also be substituted by the residue Ar;
Ar: phenyl residue which is substituted by the residues R₆, R₇ and/or R₈, wherein these residues R₆, R₇ and R₈ are identical or different and C₁-C₆ alkyl, C₃-C₇ cycloalkyl, hydroxy, C₁-C₆ alkoxy, C₂-C₆ alkanoyloxy, halogen, hydroxy, C₁-C₆ halogenoalkyl, -CN, -NH₂, -NH-C₁-C₆ alkyl, -N(C₁-C₆ alkyl)₂, -CO₂H, -CO-C₁-C₆ alkyl, -CO-O-C₁-C₆ alkyl, -COAr, -CO-OAr, -CONH₂, -CONH-C₁-C₆ alkyl, -CON(C₁-C₆ alkyl)₂, -CONHAr, -NH-CO-C₁-C₆ alkyl, -NHCO-Ar, NCHO-C₁-C₆ alkoxy, -NH-CO-OAr, -NHCO-NH₂, -NHCO-N(C₁-C₆ alkyl)₂, -NHCO-NHAr, -NH-SO₂-C₁-C₆ alkyl, -NH-SO₂Ar, -NH-SO₂-nitrophenyl, -SO₂-OH, -SO₂-C₁-C₆ alkyl, -SO₂-Ar, -SO₂-C₁-C₆ alkoxy, -SO₂-OAr, -SO₂-NH₂, -SO₂-NH-C₁-C₆ alkyl, -SO₂-N(C₁-C₆ alkyl)₂, -SO₂-NHAr, -SO₂-C₁-C₆ alkoxy;
n: 0 or 1;
and the pharmaceutically compatible acid addition salts thereof, with the exception of the compound 2-carbethoxy-amino-5-(2,4,6-trimethylbenzylamino)-aniline and the hydrochloride thereof,
characterised in that
in a manner known *per se*
a) in a compound of the formula 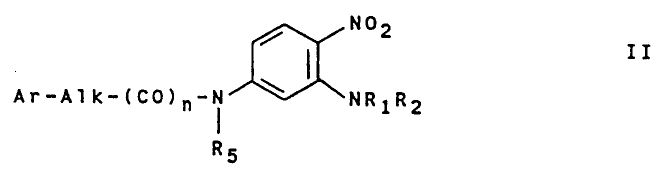 in which R₁, R₂, R₅, Alk, Ar and n have the stated meanings, or in a compound of the formula 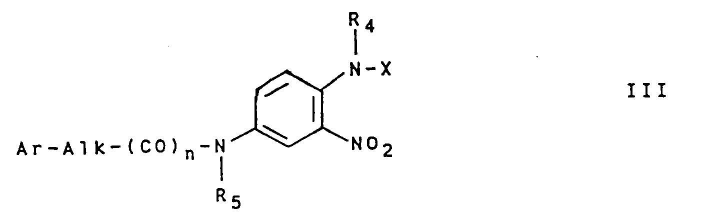 in which X is hydrogen or the group -COR₃ and R₃, R₄, R₅, Alk, Ar and n have the stated meanings,
the nitro group is reduced to the amino group, the -COR₃ group is introduced by acylation if it is not present in the starting materials II or III and, optionally after or before introduction of the -COR₃ group, the residues R₁, R₂, R₄ and R₅ are introduced by alkylation, arylation and/or acylation, or
b) a compound of the formula 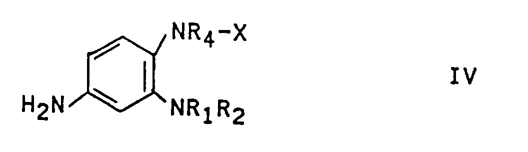 in which X is hydrogen or the group -COR₃ and R₁, R₂, R₃ and R₄ have the stated meanings is reacted with a compound of the formula
Ar-Alk-(CO)ₙHal V
in which Ar, -Alk and n have the stated meanings and Hal is chorine, bromine or iodine or with a compound of the formula
Ar-Alk'-CHO VI
in which Ar has the stated meanings and Alk' is a linear or branched alkylene group with 0 - 8 C atoms,
wherein in the latter case the double bond of the resultant Schiff's base is reduced to the single bond, the residues -COR₃, R₁, R₂, R₄ and R₅ are optionally introduced by alkylation, arylation and/or acylation, the residue R₃ in the compounds obtained using methods a) or b) is optionally exchanged for another residue falling within the stated definition for R₃ and the compounds so obtained are converted into acid addition salts.

3. Pharmaceutical preparation containing as active ingredient at least one 1,2,4-triaminobenzene derivative of the general formula 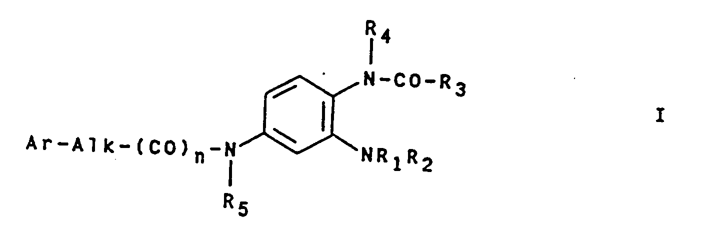 in which the symbols R₁, R₂, R₃, R₄, R₅, Ar and Alk have the following meanings:
R₁: hydrogen, C₁-C₆ alkyl, C₂-C₆ alkanoyl or the residue Ar;
R₂: hydrogen or C₁-C₆ alkyl;
R₃: C₁-C₆ alkoxy, NH₂, C₁-C₆ alkylamino, C₁-C₆ dialkylamino, amino which is substituted by the residue Ar, C₁-C₆ alkyl, C₂-C₆ alkenyl, C₂-C₆ alkynyl, the residue Ar or the residue ArO-;
R₄: hydrogen, C₁-C₆ alkyl or the residue Ar;
R₅: hydrogen, C₁-C₆ alkyl or the residue Ar;
Alk: a linear or branched alkylene group with 1-9 C atoms, which may also be substituted by the residue Ar;
Ar: phenyl residue which is substituted by the residues R₆, R₇ and/or R₈, wherein these residues R₆, R₇ and R₈ are identical or different and C₁-C₆ alkyl, C₃-C₇ cycloalkyl, hydroxy, C₁-C₆ alkoxy, C₂-C₆ alkanoyloxy, halogen, hydroxy, C₁-C₆ halogenoalkyl, -CN, -NH₂, -NH-C₁-C₆ alkyl, -N(C₁-C₆ alkyl)₂, -CO₂H, -CO-C₁-C₆ alkyl, -CO-O-C₁-C₆ alkyl, -COAr, -CO-OAr, -CONH₂, -CONH-C₁-C₆ alkyl, -CON(C₁-C₆ alkyl)₂, -CONHAr, -NH-CO-C₁-C₆ alkyl, -NHCO-Ar, NCHO-C₁-C₆ alkoxy, -NH-CO-OAr, -NHCO-NH₂, -NHCO-N(C₁-C₆ alkyl)₂, -NHCO-NHAr, -NH-SO₂-C₁-C₆ alkyl, -NH-SO₂Ar, -NH-SO₂-nitrophenyl, -SO₂-OH, -SO₂-C₁-C₆ alkyl, -SO₂-Ar, -SO₂-C₁-C₆ alkoxy, -SO₂-OAr, -SO₂-NH₂, -SO₂-NH-C₁-C₆ alkyl, -SO₂-N(C₁-C₆ alkyl)₂, -SO₂-NHAr, -SO₂-C₁-C₆ alkoxy;
n: 0 or 1;
or the pharmaceutically compatible acid addition salts thereof, optionally together with further conventional auxiliary substances and excipients or diluents.

4. Process for the production of a pharmaceutical preparation, characterised in that at least one compound according to formula I, including the compound 2-carbethoxy-amino-5-(2,4,6-trimethylbenzylamino)aniline and the hydrochloride thereof, is processed into pharmaceutical formulations or converted into a therapeutically usable form with usual pharmaceutical excipients and/or diluents or other auxiliary substances.

5. Use of compounds of the formula 1 according to claim 1, including the compound 2-carbethoxy-amino-5-(2,4,6-trimethylbenzylamino)aniline and the hydrochloride thereof, for the production of pharmaceutical preparations.

## Revendications

1. Dérivés du 1,2,4-triaminobenzène de formule générale 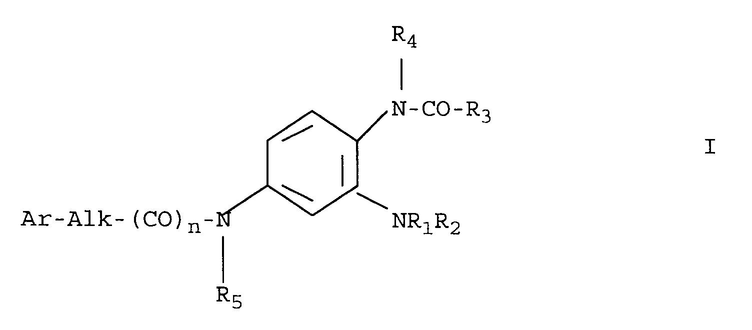 dans laquelle les symboles R₁, R₂, R₃, R₄, R₅, Ar et Alk ont les significations suivantes:
R₁: hydrogène, C₁-C₆-Alcoyl, C₂-C₆-Alcanoyl ou le radical
Ar;
R₂: hydrogène ou C₁-C₆-Alcoyl;
R₃: C₁-C₆-alcoxy, NH₂, C₁-C₆-alcoylamino, C₁-C₆-dialcoylamino, Amino, qui est substitué par le radical Ar, C₁-C₆-Alcoyl, C₂-C₆-Alcényl, C₂-C₆-Alcinyl, le radical Ar ou le radical ArO-;
R₄: hydrogène, C₁-C₆-Alcoyl ou le radical Ar;
R₅: hydrogène, C₁-C₆-Alcoyl ou le radical Ar;
Alk: un groupe alcoylène linéaire ou ramifié ayant de 1 à 9 atomes de C qui peut être aussi substitué par le radical Ar,
Ar: un radical phényle qui est substitué par les radicaux R₆ et R₇ et/ou R₈ dans lesquels les radicaux R₆, R₇ et R₈ sont identiques ou différents, et signifient; C₁-C₆-Alcoyl, C₃-C₇-Cycloalcoyl, Hydroxy, C₁-C₆-Alcoxy, C₂-C₆-Alcanoyloxy, Halogène, Hydroxy, C₁-C₆-Halogénoalcoyl, -CN, -NH₂, -NH-(C₁-C₆-Alcoyl), -N(C₁-C₆-Alcoyl)₂, -CO₂H, -CO-C₁-C₆-Alcoyl, -CO-O-(C₁-C₆-Alcoyl), -COAr, -CO-OAr, -CONH₂, -CONH-C₁-C₆-Alcoyl, -CON(C₁-C₆-Alcoyl)₂, -CONHAr, -NH-CO-(C₁-C₆-Alcoyl), -NHCO-Ar, NHCO-(C₁-C₆-Alcoxy), -NH-CO-OAr, -NHCO-NH₂, -NHCO-N(C₁-C₆-Alcoyl)₂, -NHCo-NHAr, -NH-SO₂-(C₁-C₆-Alcoyl), -NH-SO₂Ar, -NH-SO₂-Nitrophényl, -SO₂-OH, -SO₂-C(₁-C₆-Alcoyl), -SO₂-Ar, -SO₂-(C₁-C₆-Alcoxy), -SO₂-OAr, -SO₂-NH₂, -SO₂-NH-C₁-C₆-Alcoyl, -SO₂-N(C₁-C₆-Alcoyl)₂, -SO₂-NHAr, -SO₂-(C₁-C₆-Alcoxy)
n : 0 ou 1;
et leurs sels d'addition d'un acide pharmaceutiquement compatible, à l'exception du composé 2-carbethoxyamino-5-(2,4,6-triméthylbenzylamino)-aniline et de son chlorhydrate.

2. Procédé d'obtention de dérivés du 1,2,4-triaminobenzène de formule générale 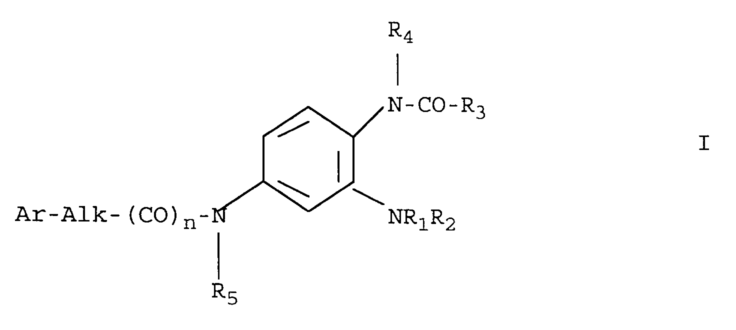 dans laquelle les symboles R₁, R₂, R₃, R₄, R₅, Ar et Alk ont les significations suivantes:
R₁: hydrogène, C₁-C₆-Alcoyl, C₂-C₆-Alcanoyl ou le radical
Ar;
R₂: hydrogène ou C₁-C₆-Alcoyl;
R₃: C₁-C₆-Alcoxy, NH₂, C₁-C₆-Alcoylamino, C₁-C₆-Dialcoylamino, amino, lequel est substitué par le radical Ar, C₁-C₆-Alcoyl, C₂-C₆-Alcényl, C₂-C₆-Alcinyl, ou le radical Ar ou le radical ArO-;
R₄: hydrogène, C₁-C₆-Alcoyl ou le radical Ar;
R₅: hydrogène, C₁-C₆-Alcoyl ou le radical Ar;
Alk: un groupe alcoylène, linéaire ou ramifié, ayant de 1 à 9 atomes de C, qui peut être aussi substitué par le radical Ar,
Ar: un radical phényle qui est substitué par les radicaux R₆ et R₇ et/ou R₈ dans lesquels les radicaux R₆, R₇ et R₈ sont identiques ou différents, et signifient; C₁-C₆-Alcoyl, C₃-C₇-Cycloalcoyl, Hydroxy, C₁-C₆-Alcoxy, C₂-C₆-Alcanoyloxy, Halogène, Hydroxy, C₁-C₆-Halogénoalcoyl, -CN, -NH₂, -NH-C₁-C₆-Alcoyl, -N(C₁-C₆-Alcoyl)₂, -CO₂H, -CO-(C₁-C₆-Alcoyl), -CO-O-(C₁-C₆-Alcoyl), -COAr, -CO-OAr, -CONH₂, -CONH-(C₁-C₆-Alcoyl), -CON-(C₁-C₆-Alcoyl)₂, -CONHAr, -NH-CO-(C₁-C₆-Alcoyl), -NHCO-Ar, NHCO-(C₁-C₆-Alcoxy), -NH-CO-OAr, -NHCO-NH₂, -NHCO-N-(C₁-C₆-Alcoyl)₂, -NHCo-NHAr, -NH-(SO₂-C₁-C₆-Alcoyl), -NH-SO₂Ar, -NH-SO₂-Nitrophényl, -SO₂-OH, -SO₂-C₁-(C₆-Alcoyl), -SO₂-Ar, -SO₂-(C₁-C₆-Alcoxy), -SO₂-OAr, -SO₂-NH₂, -SO₂-NH-(C₁-C₆-Alcoyl), -SO₂-N(C₁-C₆-Alcoyl)₂, -SO₂-NHAr, -SO₂-(C₁-C₆-Alcoxy)
n : 0 ou 1;
et leurs sels d'addition d'un acide pharmaceutiquement compatibles, à l'exception du composé 2-carbethoxyamino-5-(2,4,6-triméthylbenzylamino)-aniline et son chlorhydrate, caractérisé en ce que l'on réduit le groupe nitro d'une manière connue en soi,
a) dans un composé de formule 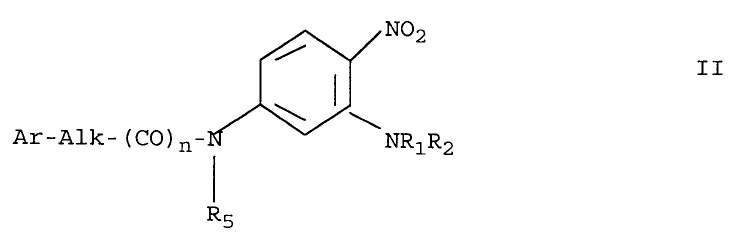 dans laquelle R₁, R₂, R₅, Alk, Ar et n ont les signification indiquées ou en un composé de formule 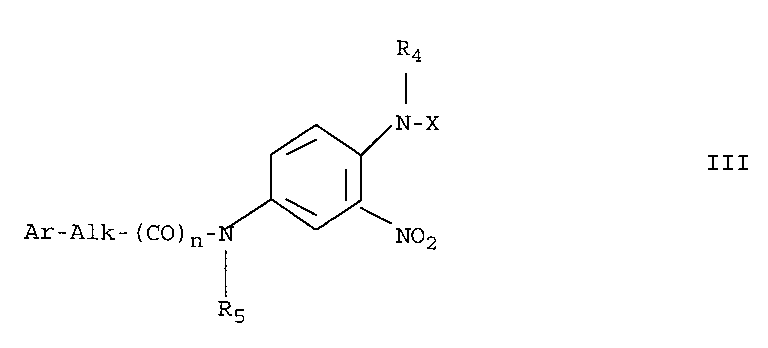 dans laquelle X est de l'hydrogène ou le groupe -COR₃ et R₃, R₄, R₅, Alk, Ar et n ont les significations indiquées, on réduit le groupe nitro en un groupe amino, on introduit par acylation le groupe-COR₃, au cas ou celui-ci n'est pas présent dans les produits de départ II ou III et on introduit le cas échéant après ou avant introduction du groupe COR₃ par alcoylation, arylation et/ou acylation les radicaux R₁, R₂, R₄, R₅ ou
b) on fait réagir un composé de formule 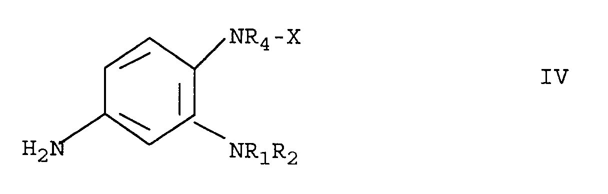 dans laquelle X est de l'hydrogène ou le groupe -COR₃ et R₁, R₂, R₃ et R₄ on les significations indiquées avec un composé de formule
Ar-Alk-(CO)ₙHal V
dans laquelle Ar, -Alk et n ont les significations indiquées et Hal est du chlore, Brome ou de l'iode ou un composé de formule
Ar-Alk'-CHO VI
dans laquelle Ar a les significations indiquées et Alk' est un groupe alcoylène linéaire ou ramifié ayant de 0 - 8 atomes de C,
dans le dernier cas la double liaison de la base de Schiff obtenue étant réduite en une liaison simple, le cas échéant par alcoylation, arylation, et/ou acylation ou introduit les radicaux -COR₃, R₁, R₂, R₄ ainsi que R₅, le cas échéant on échange dans les composés obtenus selon les procédés a) ou b) le radical R₃ contre un autre radical dans le cadre de la définition indiquée pour R₃ et on convertit les composés ainsi obtenus en sels d'addition avec un acide.

3. Médicament contenant comme principe actif au moins un dérivé de 1,2,4-triaminobenzène de formule générale 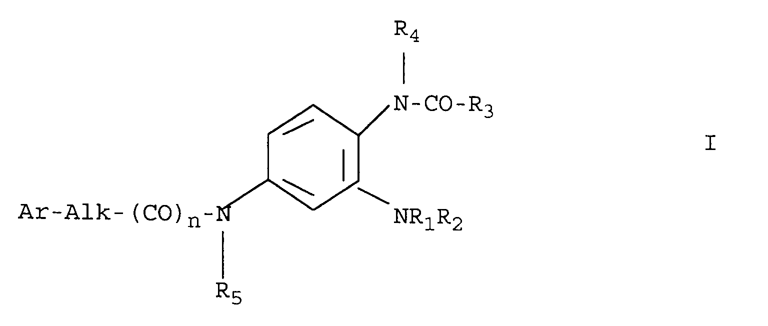 dans laquelle les symboles R₁, R₂, R₃, R₄, R₅, Ar et Alk ont les significations suivantes:
R₁: hydrogène, C₁-C₆-Alcoyl, C₂-C₆-Alcanoyl ou le radical
Ar;
R₂: hydrogène ou C₁-C₆-Alcoyl;
R₃: C₁-C₆-Alcoxy, NH₂, C₁-C₆-Alcoylamino, C₁-C₆-Dialcoylamino, amino, lequel est substitué par le radical Ar, C₁-C₆-Alcoyl, C₂-C₆-Alcényl, C₂-C₆-Alcinyl, ou le radical Ar ou le radical ArO-;
R₄: hydrogène, C₁-C₆-Alcoyl ou le radical Ar;
R₅: hydrogène, C₁-C₆-Alcoyl ou le radical Ar;
Alk: un groupe alcoylène linéaire ou ramifié ayant de 1 à 9 atomes de C qui peut être aussi substitué par le radical Ar,
Ar: un radical phényle qui est substitué par les radicaux R₆ et R₇ et/ou R₈ les radicaux R₆, R₇ et R₈ étant identiques ou différents, et signifient;
C₁-C₆-Alcoyl, C₃-C₇-Cycloalcoyl, Hydroxy, C₁-C₆-Alcoxy, C₂-C₆-Alcanoyloxy, Halogène, Hydroxy, C₁-C₆-Halogénoalcoyl, -CN, -NH₂, -NH-(C₁-C₆-Alcoyl), -N(C₁-C₆-Alcoyl)₂, -CO₂H, -CO-(C₁-C₆-Alcoyl), -CO-O-C₁-C₆-Alcoyl, -COAr, -CO-OAr, -CONH₂, -CONH-C₁-C₆-Alcoyl), -CON(C₁-C₆-Alcoyl)₂, -CONHAr, -NH-(CO-C₁-C₆-Alcoyl), -NHCO-Ar, NHCO-(C₁-C₆-Alcoxy), -NH-CO-OAr, -NHCO-NH₂, -NHCO-N(C₁-C₆-Alcoyl)₂, -NHCO-NHAr, -NH-SO₂-(C₁-C₆-Alcoyl), -NH-SO₂Ar, -NH-SO₂-Nitrophényl, -SO₂-OH, -SO₂-(C₁-C₆-Alcoyl), -SO₂-Ar, -SO₂-(C₁-C₆-Alcoxy), -SO₂-OAr, -SO₂-NH₂, -SO₂-NH-(C₁-C₆-Alcoyl), -SO₂-N(C₁-C₆-Alcoyl)₂, -SO₂-NHAr, -SO₂-(C₁-C₆-Alcoxy)
n : 0 ou 1;
ou leurs sels d'addition d'un acide comptabile pharmaceutiquement, ainsi que le cas échéant d'autres adjuvants et des supports usuels ou des agents diluants.

4. Procédé d'obtention d'un médicament, caractérisé en ce que l'on façonne au moins un composé conformément à la formule I en incluant le composé 2-carbethoxyamino-5-(2,4,6-triméthylbenzylamino)-aniline et son hydrochlorure, avec les supports et/ou les agents diluants usuels pharmaceutiquement ou d'autres adjuvants, en préparations pharmaceutiques ou on l'amene sous une forme utilisable therapeutiquement.

5. Utilisation des composés de formule I conformément à la revendication 1, tout en incluant le composé 2-carbethoxy-amino-5-(2,4,6-triméthylbenzylamino)-aniline et son hydrochlorure, pour la production de médicaments.
